# EUROPEAN PATENT APPLICATION

(11) **EP 4 144 214 A1**
(43) Date of publication of application: **08.03.2023**
(21) Application number: 21194987.0
(22) Date of filing: 06.09.2021
(51) Int. Cl.: A01K 67/033

(54) **METHOD FOR IMMOBILIZATION OF EARTHWORMS**

(71) Applicant: Bayer AG, 51373 Leverkusen (DE)
(72) Inventor: MENKE, Ulrich, 42799 Leichlingen (DE); GEHRE, Anne Claudia, 41515 Grevenbroich (DE); SUDOWE, Patrick, 58332 Schwelm (DE)
(74) Representative: BIP Patents

(57) **Abstract**

A method for immobilization of earthworms, preferably juvenile earthworms, the method comprising: Giving the earthworms into a liquid medium which comprises water and ethanol, wherein the earthworms are immobilized in the liquid medium.

## Description

### FIELD OF THE INVENTION

The present invention relates to a method for immobilization of earthworms and a method for extraction of earthworms.

### BACKGROUND OF THE INVENTION

Toxicity of chemicals entering the soil can be determined by chronic reproduction tests with Eisenia fetida/andrei. In the earthworm reproduction test (e.g. OECD 222) adult earthworms are incubated for 28 days during which they are fed and produce cocoons. After removal of the adults from the test soil, the juveniles hatching from the cocoons grow for another 28 days to be finally counted. In the untreated control samples of laboratory studies conducted with Eisenia fetida, typically between 100 and 300 juveniles are produced from 10 adults per replicate. Juvenile abundance is determined either directly by hand sorting from the soil or by extracting them by heat and picking out the individual worms when appearing at the soil surface. For a typical dose-response study with 8 concentrations (4 replicates per conc.) up to 10000 individuals need to be counted if there is no pronounced toxicity.

A usual method relies on manual counting from soil samples by searching the soil for earthworms or juveniles, and/or collecting earthworms or juveniles from the soil surface, which is time consuming and inaccurate.

A further problem is overlapping structures of earthworms or juveniles when separated from the soil which renders counting by other methods than manual methods burdensome.

### OBJECTIVE OF THE INVENTION

An objective is to develop a fixation process for earthworms, particularly juveniles. If possible, earthworms should be spread as far as possible, separated from each other as far as possible and separated from residual soil particles as far as possible and preferably be stable for some hours.

A further objective is to provide an improved counting system for juveniles.

A further objective is to develop a process for a rapid extraction of the juveniles in a clean state.

### SUMMARY OF THE INVENTION

The present invention provides a method for immobilization of earthworms, preferably juvenile earthworms, the method comprising:
Giving the earthworms into a liquid medium which comprises water and ethanol, wherein the earthworms are immobilized in the liquid medium.

In another aspect, the present invention provides a method for extraction of earthworms, preferably juvenile earthworms, from soil, the method comprising:
- Growing the earthworms within a soil, wherein the soil is comprised in a soil container having an opening, wherein the opening is closed by a lid, and the lid comprises a recess that is covered by a mesh,
- Heating the soil which causes the earthworms to move to the mesh and concentrate below the mesh, wherein at least a part of the earthworms adheres to the mesh
- removing the lid from the soil container and removing the earthworms from the mesh.

In a specific aspect, analysis of an image of earthworms is done with a trained artificial neural network in order to determine a number of earthworms. This allows automated analysis of higher number of samples, explores the advantages of automated data generation, and improves data documentation. The invention thus provides a machine learning approach, to train a system to correctly identify, label and finally count the individual earthworms. Details of the analysis method are given in the detailed description.

### DETAILED DESCRIPTION

The term "immobilization" or "immobilized" with respect to earthworms means that movement of earthworms is prevented or substantially prevented. It was found that ethanol leads to effective sedation of the earthworms.

It was also found that ethanol allows stabilization of the earthworms for some hours.

Moreover, it was found that earthworms immobilized according to the invention show reduced adherence of soil.

It was also found that soil that is still present in the liquid medium shows no or little swelling. This is beneficial for distinguishing long soil particles from earthworms because such soil particles do not reach the size of earthworms by swelling.

It was found that by the method for immobilization of the invention stretched worms could be obtained, with only a slightly curved shape. The immobilized worms look bright and transparent, which enables a good contrast to a crumbly ground. It was also found that worms less stick together. The method allows spreading the earthworms well separated from soil and from each other. This facilitates further analysis, for example of images.

The term "earthworms" means worms of the sub-class Oligochaeta, except for Enchytreids and Tubificides/Naididae, in particular worms of the family Lumbricidae. A preferred earthworm is from the family Eisenia, more preferred *Eisenia fetida* or *Eisenia andrei.*

The liquid medium is water-based. The liquid medium is, in other words, an aqueous liquid medium comprising a small-chain alcohol (referred to as alcohol), ethanol or isopropanol, preferred ethanol. The term "water-based" or "aqueous" means that the liquid medium comprises at least 50 vol.-%, preferably at least 70 vol.-%, even more preferably at least 75 vol.-% of water. The remainder (the part of the liquid medium which is not water) comprises the alcohol. The liquid medium may comprise optional further ingredients, such as below mentioned hydrochloric acid.

The liquid medium can, measured in volume units, consists or essentially consists of water and alcohol.

In an embodiment of the invention, the content of alcohol in the liquid medium is in a range of 5 to 20 vol.%, more preferably 7 to 15 vol.%, even more preferably 8 to 12 vol.%.

In an embodiment of the invention, the liquid medium further comprises an inorganic acid, most preferred hydrochloric acid (HCl). It turned out that by addition of HCl immobilization could be further improved because HCl leads to faster immobilization. Faster immobilization is beneficial for higher throughput of samples and potential automatization, particularly when data is analyzed with a trained artificial neural network as described below.

In an embodiment of the invention, the concentration of the hydrochloric acid in the liquid medium is in a range of 0.003 Mol/L to 0.3 Mol/L, preferably 0.01 Mol/L to 0.1 Mol/L. In the invention, the liquid medium may comprise ethanol and hydrochloric acid in indicated ranges and the remainder may be water or substantially consisting of water. The term "substantially consisting of water" in this regard means that at least 95-vol.-%, preferably at least 98-vol.-% of the remainder is water.

In one embodiment the pH of a liquid medium comprising the inorganic acid is pH 1 to 3.

In an embodiment of the invention, the earthworms are distributed over an inner bottom surface of a container that comprises the liquid medium. For example, the earthworms may be given into a container comprising the liquid medium. Immobilization leads to a distribution of the earthworms the over a bottom inner surface.

In a more specific embodiment, the container has a transparent bottom, which means a light transparent, or translucent, bottom. By this embodiment, the possibility of illumination of earthworms is improved, particularly for capturing an image, particularly with use of translucent light.

In an embodiment of the invention the method for immobilization further comprises:
- capturing an image of immobilized earthworms.

Usual digital photographic technique and equipment can be used for this purpose. Preferably, translucent light is used to illuminate the immobilized earthworms.

In an embodiment of the invention, the method for immobilization further comprises:
- analyzing the image using a trained artificial neural network (hereinafter also ANN) in order to determine a number of earthworms.

This is a beneficial technique in comparison to image analysis by hand or by counting earthworms manually, because counting could be automated, and higher throughput of samples can be reached.

Training data for training an ANN can be obtained, for example, by marking of earthworms against a background in a plurality of captured images, thereby obtaining segmentation masks. The ANN can be trained with such pairs of image/ corresponding segmentation mask. The trained ANN can then be used for automatic image analysis.

The ANN can be based, or work on basis of, object detection, Semantic Segmentation and/or Instance Segmentation. In Semantic Segmentation the object class to which it belongs is calculated for each pixel. In Instance Segmentation, Object Type and Object Instance are determined for all pixels.

A method for training an ANN by using pairs of image/ corresponding segmentation mask is described for example in: The U-Net: Convolutional Networks for Biomedical Image Segmentation; Olaf Ronneberger, Philipp Fischer, Thomas Brox, MICCAI 2015.

In a specific embodiment, a facilitated process is used for training an ANN. In this embodiment, the trained artificial neural network is obtained by the following:
- training an initial artificial neural network by using pairs of
   (i) images already present for structures comparable to the immobilized earthworms and
   (ii) corresponding segmentation masks for the comparable structures, thereby obtaining a trained initial artificial network,
- applying the trained initial artificial network on images of the immobilized earthworms in order to obtain training data, the training data comprising pairs of
   (I) images of the immobilized earthworms, and
   (II) corresponding estimated segmentation masks for the immobilized earthworms, 125 estimated by the trained initial artificial network,
- training a second artificial neural network with the training data in order to obtain the trained artificial neural network.

This can also be described as counting in two steps, thereby avoiding instance segmentation which requires a lot of data:
a) Semantic segmentation - deep learning: A first Artificial Neural network is trained from images and manually created masks, including masks that were generated on another data set. Thereby an initial data set is generated.
b) The initial data set is used to train a second ANN and then the trained second ANN is applied to new images of immobilized earthworms to obtain an estimated segmentation.

This embodiment leads to good results without labeling. Less training data (images) of immobilized earthworms is needed. Training can be done according to the method published by: Convolutional Networks for Biomedical Image Segmentation; Olaf Ronneberger, Philipp Fischer, Thomas Brox, MICCAI 2015.

The second artificial neural network may have the same architecture or structure than the initial artificial neural network.

Obtained corresponding estimated segmentation masks for the immobilized earthworms may be filtered using the anisotropy and size of the connected component found.

Comparable structures are, for example, other kinds of worms with comparable structure. Preferable Comparable structures are in the present invention Caenorhabditis Elegans (C. Elegans). Data of C. Elegans could be obtained from: C. elegans live/dead assay | Broad Bioimage Benchmark Collection (broadinstitute.org; https://bbbc.broadinstitute.org/BBBC010).

In a further aspect, the method for immobilization of earthworms further comprises identifying overlapping earthworms in the image of immobilized earthworms. This is preferably done by conventional image processing. Identification of overlapping earthworms is preferably used to improve the counting result obtained after image analysis with a trained ANN. Identifying overlapping earthworms can be based on counting by tracing. Identifying overlapping earthworms can comprise one or more of the following:
a. Graph reduction
b. End points - preferably rule-defined - may follow a smooth / straight structure. Assumptions may be predefined, e.g. no sharp angles, no 90 degrees. The smoothest lines are continued from all connections. The straightest continuation may be selected at intersections.
c. Intermediate points are recognized
d. Organisms are counted
e. Output of the recognized organisms and their number

In a further embodiment, the method for immobilization further comprises, before giving the earthworms into the liquid medium:
- Growing the earthworms within a soil, wherein the soil is comprised in a soil container having an opening, wherein the opening is closed by a lid, and the lid comprises a recess that is covered by a mesh;
- Heating the soil which causes the earthworms to move to the mesh and concentrate below the mesh, wherein at least a part of the earthworms adheres to the mesh;
- removing the lid from the soil container and removing the earthworms from the mesh.

This embodiment is a combination of the method of the invention for immobilization of earthworms and the method of the invention for extraction of earthworms (cf. Summary). All aspects concerning this embodiment are also applicable to the general independent method of the invention for extraction of earthworms.

The mesh has openings with an area smaller than the cross section (area) of the earthworms, particularly earthworm juveniles, so that earthworms are retained. In one embodiment, the mesh is a gauze.

The mesh is permeable to air. The worms are driven up by the temperature and collect under the mesh, because this is where the temperature is lowest due to evaporation. The mesh allows for air exchange but prevents earthworms to leave. Only here the warm and humid air above the soil could spew out of the vessels and condensate above the vessel.

Preferably there is a gap between the surface of the soil and the mesh. This allows the earthworms to concentrate in the gap, preferably as agglomerate or pile.

In this embodiment, earthworms are attached to the mesh and could be manually collected. Earthworms are preferably present in a pile or agglomerate that is attached to the mesh. So, a relatively high number of earthworms could be collected at once, by removing the pile from the mesh.

In one embodiment of the method, the recess is located in a central position on the lid, or in other words centered on the lid.

After removing the earthworms from the mesh, the earthworms can be immobilized as described above.

This embodiment allows extraction of earthworms from artificial soil in relatively clean form. No or only a few adherence of soil, particularly kaolin, has been observed.

The soil may be heated to a temperature in the range of 40 - 70°C, preferably 50 - 60°C. The time of heating may be 15 to 60 min, preferably 20 to 50 min

### BRIEF DESCRIPTION OF THE DRAWINGS

Fig. 1 shows a device for extraction of earthworms which is used in a method of the present invention for extraction of earthworms;
Fig. 2 shows an image of immobilized earthworms prepared according to a method immobilization of the present invention;

### References:

soil container 1
soil 2
opening 6
lid 3
recess 4
mesh 5
5a overlapping region of mesh 5 and lid 3
7 earthworms
10 inner bottom surface of the transparent container for image acquisition

Fig. 3 shows a process scheme for image analysis according to the present invention.

### EXAMPLES

### Example 1: Growing earthworms and extraction from soil

### Test Design: Dose-response test (OECD 222)

For the ease of experiment artificial soil, for example 10% peat, 70% sand, 20% Kaolin, was used.
- Test item is mixed into the soil, food weekly: cow manure
- 8 concentrations (dose-response), 4 replicates/concentration
- 10 adult earthworms per test vessel from a synchronized culture, weight 300 - 600 mg
- Phase 1 (days 0-28): adult worms present
=> production of cocoons, hatching, juveniles

The person skilled in the art will appreciate that the method is expected to be usable for any soil, artificial and natural soils.
- Phase 2 (days 28-56): adults taken out, further growth of juveniles w/o additional food to a size they are well observable

### Evaluation:

Test vessels (soil contained) (16.5 x 12 cm -Bellaplast vessels with 500 g dw soil) containing the juvenile earthworms in a 5 cm layer of soil were heated from below in a water bath of about 55 °C (The depth of the water at the edge of the Bellaplast vessels is approx. 2 cm) for about 35 min forcing the juveniles to move to the soil surface. Special lids were constructed that had a central mesh (ca. 4 cm x 4 cm) allowing for air exchange but preventing juveniles to leave. Only here the warm and humid air above the soil could spew out of the vessels and condensate above the vessel. The juveniles accumulated after 40 minutes nearly completely below the mesh since this was the coldest point and could be taken out completely as a single agglomerate with only few soil particles attached to them.

The necessary movement of the earthworms simultaneously guarantees that all sampled animals are alive.

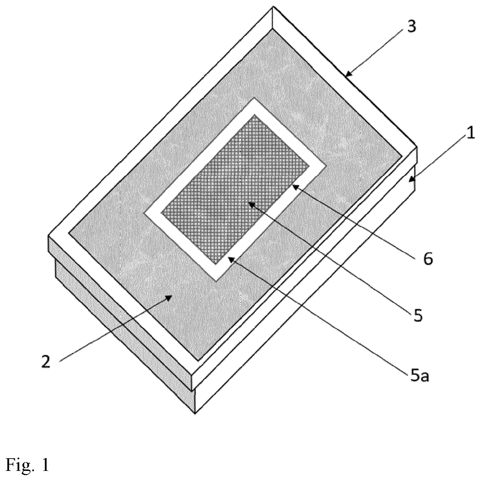

Fig. 1 shows the device used for extraction of earthworms. The device comprises the soil container 1 that is filled with soil 2. The soil container has the opening 6 that is closed with the lid 3. The lid 3 comprises the recess 4 that is covered by a mesh 5. The region 5a shows an overlapping region of mesh 5 and lid 3. It is shown that the whole recess 4 is covered by the mesh 5.

### Example 2: Fixation medium

Several solutions of different substances, solvents and pH values were tested to fix the earthworms taken from the soil. The juveniles were transferred to large petri dishes containing 200 mL of this mixture (Fully transparent PET vessel 25 x 25 [500 cm2], 2 cm high).

Media Testing - the results of the media testings are summarized in Table 1 to 3.

Criteria for evaluation were as follows:
- Immobilization of juveniles
   Form of juveniles: If possible, stretched (not contracted)
- Similarity of juvenile's form to each other
- Form of soil particles
- Separation of soil and juveniles
- Separation of juveniles from each other
- Separation of juveniles from walls of PET vessel
- Crossing of juveniles with each other and themselves
- Contrast of colors between juveniles and soil particles
- Difference of forms / color of soil and juveniles
- Stability of the appearance after some time of incubation
- Reproducibility of appearance

**Table 1**

| **Criteria evaluated** | **Glycerin 50% or 33%** | **Tap water** | **Detergent*** | **NaCl solution 5%** | **Sucrose 10%** | **Ethanol 10%** |
|---|---|---|---|---|---|---|
| Immobilization of juveniles (juv.) | after 2 min | no | after ca. 10 min | immediately | slow movements, some jerking | after 3 min |
| Form of juv. | strongly contracted, short, thick, uneven form | more stretched | more stretched | slightly contracted, even | more stretched, some coiled at and | stretched with characteristic Boomerang or (small juv.) hook form after 60 min |
| Similarity of juv. forms to each other | extremely different | medium | medium | better | extremely different | high |
| Form of soil particles | very different in size and form | very fluffy, different to each other | less fluffy, a bit less different to each other | very different, sand separates, clotty | very different, sand separates, clotty | fine structured, relatively similar to each other |
| Separation of soil and juv. | ○ | + | + | - | -- | ++ |
| Separation of juv. from each other | ○ | ○ | + | - | ○ | + |
| Separation of juv. from walls of PET vessel | + | ○ | ○ | ○ | ○ | + (due to form) |
| Crossing of juv. with each other | ○ | - | ○ | ○ | ○ | ++ |
| Crossing of juv. with themselves | - | ○ | ○ | ○ | - | ○ |
| Contrast of colors betweenjuv. and soil particles and background | worms light to very dark, soil dark, very small juv: difficult to see | worms light to structured dark, soil dark, very small juv: partially difficult to see | worms light to structured dark, soil dark, very small juv: partially difficult to see | worms and soil particles equally dark, no contrast, worms not structured | smaller worms: light (and structured), biggerworms: dark | worms of all sizes lighter and well structured, soil particles dark: very good contrast! |
| Difference of forms of soil and juv. | Juv. difficult to distinguish | Juv. OK to be distingui shed | Juv. OK to be disting uished | small juv. not so easy to be distinguished | Juv. OK to be distinguished | Juv. very easy to be distinguished from soil particles |
| Stability/Suitability of the appearance after some time of incubation | + | - | - | not checked | - | ++ |
| Reproducibility of appearance | + | + | ○ | not checked | not checked | + |
| Suitability | - | ○ | + | - | - | ++ |
| Comment/Conclusion | Difficult to distinguish | OK, but no immobilization, soil particles very different, very small juv. difficult to see | OK, but not so reproducib le, very smalljuv. difficult to see | No contrast between soil and worms | Strong clotting of earthworms to each other | Most suitable: very characteristic form and light well-structured appearance: best after waiting for > 1 hour |

| | | | | | | |
|---|---|---|---|---|---|---|
| * **Dishwashing, 1 droplet** | | | | | | |

**Table 2**

| **Criteria evaluated** | **Ethanol 20%** | **2-Propanol 10%** | **Ethanediol 20%** | **Acetone 5%** |
|---|---|---|---|---|
| Immobilization of juveniles (juv.) | after 2 min | after 2 min | Initially strongly activated, immobile after ca. 10 min | Strongly activated, still mobile after 20 min |
| Form of juv. | Less stretched / more curved form than 10% Ethanol | Strongly curved to a full circle. Partial stretching after several hours but less stretched at mouth end | Initially contracting, shrinkling after 20 min to thick uneven form | slightly contracted to slightly stretched, extremely dissimilar forms |
| Similarity of juv. forms to each other | Medium | medium | extremely different | extremely different |
| Form of soil particles | Mostly smaller, partially bigger particles | Soil clumps together forming big and uneven particles | soil particles clumping together to very different sized particles | soil particles clumping together |
| Separation of soil andjuv. | ++ | - | -- | - |
| | | (big soil conglomerates sticks partially to juv.) | (soil strongly adhering to juveniles) | (soil adhering to juveniles) |
| Separation of juv. from each other | + | + | ○ | ○ |
| Separation of juv. from walls of PET vessel | + (due to form) | + | + | + |
| Crossing of juv. with each other | + | ○ | ○ | ○ |
| Crossing of juv. with themselves | + | + | + | + |
| Contrast of colors between juv. and soil particles and background | + (juv. are transparent, gut content visible) | + | - | - |
| Difference of forms of soil and juveniles | ++ Juv. very easy to distinguish from soil particles (after 4 h) | ++ Juv. very easy to distinguish from soil particles | + | + |
| Stability/Suitability of the appearance after some time of incubation | o (stretching ongoing overnight) | + | n.d. | - (shape of juv. changing strongly over time) |
| Reproducibility of appearance | + | n.d. | n.d. | n.d. |
| Suitability | ++ | - | - | - |
| Comment/Conclusion | Good, but more curved shape less suitable than 10% Ethanol, period needed for stretching is much longer | Not suitable for easy image analysis | Not suitable for easy image analysis | Not suitable for easy image analysis |

**Table 3**

| **Criteria evaluated** | **Ethanol 10% (10 min preinc.) and 0.02% Sirius Red (50 min)** | **Ethanol 20% (10 min preinc.), add of 0.02% Sirius Red* in HCl pH 2 (50 min)** | **Ethanol 10% in HCl 0.03 N (direct mix)** | **Ethanol 20% (10 min preinc.), add of 0.02% Alcian Blue** in 3% acetic acid (50 min)** | **Ethanol 10% in 3% acetic acid (direct mix)** |
|---|---|---|---|---|---|
| Immobilization of juveniles (juv.) | After 3 min | After 2 min | Instantly after few seconds | After 2 min | Instantly after 1 min |
| Form of juv. | Nicely stretched long and more curved boomerang shape. Juv. colored in dark red. Red background color in medium. | Partially stretched slightly curved to strongly curved (no circles). Juv. lightly colored in red. Soil colored. Slight red background color and precipitated as very small particles | Nicely stretched long and only slightly curved boomerang shape after 30 min. | Curved up to half circles, stable after 50 min. Juv. stained in light blue. Soil particles also blue. Blue background color evenly distributed. | Curved up to half circles, stable after 50 min |
| Similarity of juv. forms to each other | medium | medium | high | better | better |
| Form of soil particles | Soil fluffy - sticks to itself with time, particles growing over time | Fine small particles (few bigger particles, uneven) | Fine small particles (few bigger particles, uneven) | Fine small particles (few bigger particles, uneven) | Fine small particles (few bigger particles, uneven) |
| Separation of soil and juv. | Soil strongly sticks to juv. | o (soil sticks partially to juv.) | ++ | o (soil sticks partially to juv.) | ○ (soil sticks partially to juv.) |
| Separation of juv. from each other | ○ | + | + | ○ | ○ |
| Separation of juv. from walls of PET vessel | + | + | + (due to form) | ++ (due to form) | ++ (due to form) |
| Crossing of juv. with each other | + | ○ | + | ○ | ○ |
| Crossing of juv. with themselves | + | + | ++ | + | ○ |
| Contrast of colors between juv. and soil particles and background | + | + | + (juv. are transparent, gut content visible) | + | ○ |
| Difference of forms of soil and juveniles | + | + | ++ Juv. very easy to distinguish from soil particles | ++ Juv. very easy to distinguish from soil particles | ++ Juv. very easy to distinguish from soil particles |
| Stability/Suitability of the appearance after some time of incubation | ○ | n.d. | ++ (stable overnight) | + | + |
| Reproducibility of appearance | - | + | + | + | n.d. |
| Suitability | - | + | +++ | + | + |
| Comment/Conclusion | Not suitable for easy image analysis (soil strongly attached to juv.) | OK, but color gives no added value, creating background * for staining of earthworm collagenous outer cuticle | Best results. Stretching accelerated and more even than with 10% Ethanol alone. Direct mixing of Ethanol and HCl before adding to earthworms is preferred. | OK, but color gives no big added value, * for staining of earthworm epidermal mucous cells | OK, but partially sticking soil particles |

A combination of hydrochloric acid (0.03 N) with Ethanol (10%) was found to be best mode. Within 15-30 min they were stretched and turned to a characteristic color and curved shape. They appeared transparent, the dark gut content being clearly visible. This state of the juveniles was stable overnight. The individuals were mostly separated well from each other and from residual soil particles. However, some overlapping of individual juveniles with each other was apparent.

### Example 3: Taking Images

High resolution photographic images were taken by a Digital Reflex (SLR) camera using a repro stand and illumination of the transparent PET vessel from below. Parameters: 1/25; Aperture 7.1; ISO 100, focal length 18 mm.

### Example 4: Identification and Counting

An automatic image segmentation method based on deep neural networks was utilized to identify and count the juvenile earthworms. We utilized an automatic image segmentation method based on deep neural networks, which have been pre-trained on publicly available grayscale images with labeled nematodes to detect worm like structures. This deep neural network was then refined in combination with additional filtering methods to segment earthworms in a previously unseen color image dataset.

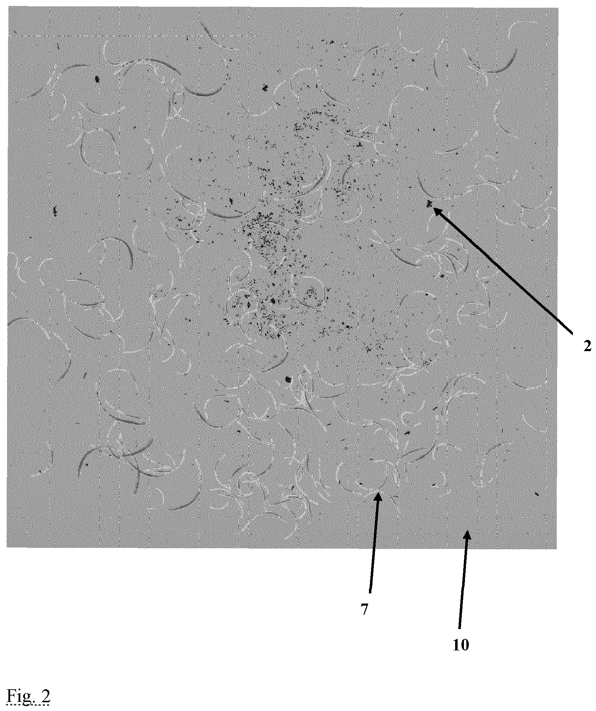

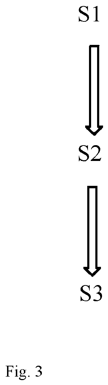

The image segmentation method is shown in Fig. 3 in following steps:
Step S1: training an initial artificial neural network by using pairs of
   (i) images already present for structures comparable to the immobilized earthworms and
   (ii) corresponding segmentation masks for the comparable structures, thereby obtaining a trained initial artificial network.
Step S2: applying the trained initial artificial network on images of the immobilized earthworms in order to obtain training data, the training data comprising pairs of
   (I) images of the immobilized earthworms (such as for example shown in Fig. 2), and
   (II) corresponding estimated segmentation masks for the immobilized earthworms.
Step S3: training a second artificial neural network with the training data in order to obtain the trained artificial neural network.

The earthworms were successfully identified and detected independent of their differing sizes. In few cases the segments were not perfectly detected resulting in two separate juveniles instead of a single worm or only partial labeling of a single longer earthworm.

A further analysis is directed to identification and separation of overlapping structures, which is done by conventional image processing without labeling.

A tracing method (graph algorithm) is used for identification and counting.

The tracing algorithm used was as follows:
Input: Connected Components of image Pixels belonging to a group of worms, sampling radius r Ouput: Estimated Number of Worms
1. Skeletonization: Compute Skeleton of Pixel group with skimage.morphology.skeletonize (https://scikit-image.org/docs/dev/api/skimage.morphology.html#skimage.morphology.skeletonize)
   The Skeleton describes the Graph structure along which the tracing/sampling is performed
2. Compute all endpoints of the graph
3. Keep priority list of current vertex to continue tracing: Fill priority list with endpoints The priority is computed based on the curvature of the line segment in the sampling radius
   a) Sample all points with distance of radius r that are reachable over the graph connectivity
   b) Compute curvature and add it to the priority list
4. Pick highest priority point and continue tracing and filling the priority list until the priority list is empty.

## Claims

1. A method for immobilization of earthworms, preferably juvenile earthworms, the method comprising:
Giving the earthworms into a liquid medium which comprises water and ethanol, wherein the earthworms are immobilized in the liquid medium.

2. The method of claim 1, wherein the content of ethanol in the liquid medium is in a range of 5 to 20 vol.%.

3. The method of claim 1, wherein the content of ethanol in the liquid medium is in a range of 7 to 15 vol.%.

4. The method of one or more of the preceding claims, wherein the liquid medium further comprises hydrochloric acid.

5. The method of claim 4, wherein the concentration of the hydrochloric acid in the liquid medium is in a range of 0.003 Mol/L to 0.3 Mol/L.

6. The method of claim 5, wherein the concentration of the hydrochloric acid in the liquid medium is in a range of 0.01 Mol/L to 0.1 Mol/L.

7. The method of one or more of the preceding claims, wherein the earthworms are distributed over an inner bottom surface of a container that comprises the liquid medium.

8. The method of claim 7, wherein the container has a transparent bottom.

9. The method of one or more of the preceding claims, the method further comprising: capturing an image of immobilized earthworms.

10. The method of claim 9, the method further comprising:
- analyzing the image using a trained artificial neural network in order to determine a number of earthworms.

11. The method of claim 10, wherein the trained artificial neural network is obtained by the following:
- training an initial artificial neural network by using pairs of
(i) images already present for structures comparable to the immobilized earthworms and
(ii) corresponding segmentation masks for the comparable structures, thereby obtaining a trained initial artificial network,
- applying the trained initial artificial network on images of the immobilized earthworms in order to obtain training data, the training data comprising pairs of
(I) images of the immobilized earthworms, and
(II) corresponding estimated segmentation masks for the immobilized earthworms,
- training a second artificial neural network with the training data in order to obtain the trained artificial neural network.

12. The method of claim 11, further comprising identifying overlapping earthworms in the image of immobilized earthworms.

13. The method of one or more of the preceding claims, the method further comprising, before giving the earthworms into the liquid medium
- Growing the earthworms within a soil, wherein the soil is comprised in a soil container having an opening, wherein the opening is closed by a lid, and the lid comprises a recess that is covered by a mesh;
- Heating the soil which causes the earthworms to move to the mesh and concentrate below the mesh, wherein at least a part of the earthworms adheres to the mesh;
- removing the lid from the soil container and removing the earthworms from the mesh.

14. The method of claim 13, wherein the recess is located in a central position on the lid.

15. A method for extraction of earthworms, preferably juvenile earthworms, from soil, the method comprising:
- Growing the earthworms within a soil, wherein the soil is comprised in a soil container having an opening, wherein the opening is closed by a lid, and the lid comprises a recess that is covered by a mesh,
- Heating the soil which causes the earthworms to move to the mesh and concentrate below the mesh, wherein at least a part of the earthworms adheres to the mesh
- removing the lid from the soil container and removing the earthworms from the mesh
